# EUROPÄISCHE PATENTSCHRIFT

(11) **EP 4 103 148 B1**
(45) Veröffentlichungstag und Bekanntmachung des Hinweises auf die Patenterteilung: **22.01.2025**
(21) Anmeldenummer: 21701684.9
(22) Anmeldetag: 14.01.2021
(51) Int. Cl.: A61K 8/73, A61K 8/06, A61Q 17/04

(54) **POLYACRYLAT-FREIE KOSMETISCHE ZUBEREITUNG**
COMPOSITION FREE FROM POLYACRYLATE
COMPOSITION NE COMPRENANT PAS DE POLYACRYLATES

(30) Priorität: 13.02.2020 DE 102020201799
(43) Veröffentlichungstag der Anmeldung: 21.12.2022
(73) Patentinhaber: Beiersdorf AG, 22529 Hamburg (DE)
(72) Erfinder: SCHLENKER, David, 22607 Hamburg (DE); VON DER FECHT, Stephanie, 22880 Wedel (DE); VOLBRICH, Heike, 22303 Hamburg (DE); SPROCK, Sarah, 22297 Hamburg (DE)
(74) Vertreter: Beiersdorf AG
(86) Internationale Anmeldenummer: PCT/EP2021/050661
(87) Internationale Veröffentlichungsnummer: WO 2021/160366

(56) Entgegenhaltungen:
- EP-A1- 3 351 236
- EP-A2- 2 837 407
- DE-A1- 102017 202 838
- DATABASE GNPD [online] MINTEL; 20 January 2020 (2020-01-20), ANONYMOUS: "Organic Aloe White Sunblock SPF 30", XP055798098, retrieved from https://www.gnpd.com/sinatra/recordpage/7198509/ Database accession no. 7198509
- DATABASE GNPD [online] MINTEL; 26 November 2018 (2018-11-26), ANONYMOUS: "Lifting & Firming Day Cream with White Pearl SPF 15", XP055798101, retrieved from https://www.gnpd.com/sinatra/recordpage/6148957/ Database accession no. 6148957
- DATABASE GNPD [online] MINTEL; 26 July 2016 (2016-07-26), ANONYMOUS: "Fluid Lotion Very High Protection SPF 50+", XP055798211, retrieved from https://www.gnpd.com/sinatra/recordpage/4167937/ Database accession no. 4167937
- DATABASE GNPD [online] MINTEL; 22 April 2013 (2013-04-22), ANONYMOUS: "Anti-Ageing Cream", XP055798212, retrieved from https://www.gnpd.com/sinatra/recordpage/2021948/ Database accession no. 2021948
- VIACHEM: "XANTHAN GUM IN COSMETICS AND TOILETRIES", 1 June 2018 (2018-06-01), XP055798249, Retrieved from the Internet <URL:https://viacheminc.com/wp-content/uploads/2018/06/Information-Sheet-Xanthan-Gum-in-Cosmetics-and-Toiletries.pdf> [retrieved on 20210422]

## Beschreibung

Die vorliegende Erfindung betrifft eine kosmetische Zubereitung enthaltend Hydroxypropylmethylcellulose und Xanthangummi, wobei die Zubereitung frei ist von Polyacrylaten, Carbomeren und Polyvinylpyrrolidonen, sowie Verfahren und Verwendungen von Hydroxypropylmethylcellulose und Xanthangummi in Polyacrylat-freien kosmetischen Zubereitungen

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurde daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Eine weit verbreitete Nutzungsform kosmetischer Sonnenschutzmittel findet rund um den Wassersport statt. Gerne werden die Sonnenschutzmittel in Verbindung mit Strandurlaub, Besuchen in Schwimmbädern, beim Baden, Surfen, Tauchen, Paddeln oder anderen Aktivitäten eingesetzt, bei denen die menschliche Haut mit Wasser in Kontakt kommt.

Bei allen diesen Aktivitäten kommt es für einen umfassenden UV-Schutz darauf an, dass das Sonnenschutzmittel eine gewisse Wasserfestigkeit aufweist und nicht sofort bei Kontakt mit dem Wasser von der Haut abgespült wird.

Zur Erhöhung der Wasserfestigkeit kosmetischer Sonnenschutzmittel werden diesen Zubereitungen in der Regel sogenannte Filmbildner zugesetzt. Bei den Filmbildnern handelt es sich üblicherweise um polymere Verbindungen auf der Basis von Polyacrylaten oder Polyvinylpyrrolidonen, die auf der Haut einen Schutzfilm ausbilden, der das Abwaschen der UV-Filter von der Haut verhindern/verzögern soll.

Polyacrylate werden darüber hinaus als Ersatz-Stoff für niedermolekulare Emulgatoren eingesetzt. Bei Ihnen beruht die emulgierende Wirkung auf der Ausbildung eines Gelnetzwerkes in der Zubereitung, welches die Öltröpfchen in der wässrigen Umgebung stabilisiert und ein Zusammenfließen derselben verhindert.

Der Einsatz derartiger Polymere wird von einer breiten Öffentlichkeit zunehmend kritisch gesehen. Es wird in der öffentlichen Diskussion des Öfteren die Sorge geäußert, derartige Polymere können analog dem so genannten "Mikroplastik" eine Belastung für die Umwelt darstellen. Ob derartige Sorgen berechtigt und naturwissenschaftlich begründet sind, kann im Rahmen der vorliegenden Offenbarung dahingestellt bleiben. Tatsache ist hingegen, dass bei den Kosmetikherstellern und Verbrauchern zunehmend ein Interesse daran besteht, alternative Techniken zur Gewährleistung der Wasserfestigkeit und Emulsionsstabilität von Sonnenschutzmitteln und anderen Kosmetika zu entwickeln. Die Zubereitungen sollen nach dem Verständnis der Verbraucher "Mikroplastik-frei" sein.

Es war daher die Aufgabe der vorliegenden Erfindung, ein möglichst wasserfestes Sonnenschutzmittel zu entwickeln, bei dem auf den Einsatz von polymeren Filmbildnern (insbesondere solchen auf Basis von Polyacrylaten und Polyvinylpyrrolidonen) weitgehend verzichtet werden kann. Es war ferner die Aufgabe der vorliegenden Erfindung, eine kosmetische

Zubereitung zu entwickeln, bei dem auf den Einsatz von synthetischen Polymeren und insbesondere solchen auf Basis von Polyacrylaten verzichtet werden kann.

Überraschend gelöst wird die Aufgabe durch eine kosmetische Zubereitung enthaltend
a) Hydroxypropylmethylcellulose und
b) Xanthangummi,

wobei die Zubereitung frei ist von Polyacrylaten, Carbomeren und Polyvinylpyrrolidonen,
dadurch gekennzeichnet, dass das Gewichtsverhältnis von Hydroxypropylmethylcellulose zu Xanthangummi in der Zubereitung von 1:2 bis 2:7 beträgt.

Überraschend gelöst wird die Aufgabe ferner durch ein Verfahren zur Viskositätsregulierung Polyacrylat-freier kosmetischer Zubereitungen, dadurch gekennzeichnet, dass der Zubereitung eine Mischung aus Hydroxypropylmethylcellulose und Xanthangummi zugesetzt wird.

Überraschend gelöst wird die Aufgabe nicht zuletzt durch die Verwendung einer Mischung aus Hydroxypropylmethylcellulose und Xanthangummi als Ersatzstoff für Polyacrylate in Polyacrylat-freien kosmetischen Zubereitungen.

Zubereitungen mit der erfindungsgemäßen Kombination an Inhaltsstoffen weisen gegenüber anderen Zellulosen und Gummen eine höhrere Temperatur- und Lagerstabilität auf. Während beim Einsatz anderer natürlicher Verdicker wie Hydroxpropyl Starch Phosphate, Microcristalline Cellulose oder Gellan Gum nach längerer Lagerzeit bzw. erhöhter Lagertemperatur Phasentrennungen (Ölabscheidungen) zu beobachten waren, erwies sich die erfindungsgemäße Stoffkombination als erstaunlich stabil.

Die Formulierungen "erfindungsgemäß", "erfindungsgemäß vorteilhaft" etc. beziehen sich im Rahmen der vorliegenden Offenbarung immer auf die erfindungsgemäße Zubereitung, die erfindungsgemäße Verwendung und das erfindungsgemäße Verfahren, wenn es im Einzelfall nicht anders beschrieben wird.

Zwar kennt der Stand der Technik die Datenbankeinträge in der Mintel Datenbank GNPD mit den Registrierungsnummern Nr. 7198509 ("Organic Aloe White Sunblock SPF 30"), Nr. 6148957 ("Lifting & Firming Day Cream with White Pearl SPF 50+") und Nr. 4167937 ("Anti-Agening Cream"), sowie den Aufsatz "Xanthan Gum in Cosmetics and Toiletries von Viachem und die Patentanmeldung DE 102017202838 A1, doch konnten diese Veröffentlichungen nicht den Weg zur vorliegenden Erfindung weisen.

Als UV-Filter gelten erfindungsgemäß alle Substanzen, die in der Verordnung (EU) Nr. 866/2014 und der Verordnung Nr. 1223/2009 der EU Kommission in der Anlage VI als UV-Filter aufgelistet sind.

Als Hydroxypropylmethylcellulose wird erfindungsgemäß vorteilhaft eine Verbindung eingesetzt, die ein mittleres Molekulargewicht von 80000 bis 90000 g/mol aufweist. Erfindungsgemäß bevorzugt weist eine 2%ige wässrige Lösung der Hydroxypropylcellulose bei 20 °C eine Viskosität von 400 cPs auf. Erfindungsgemäß vorteilhaft kann beispielsweise Methocel E4M Premium von Dow Chemical oder Tylose E707002 von SE Tylose GmbH & Co. KG eingesetzt werden.

Als Xanthangummi kann erfindungsgemäß vorteilhaft eine Verbindung eingesetzt werden, die 1 %ig in einer 1%igen KCI-Lösung unter Standardbedingungen bei 60 rpm eine Viskosität von 1200-1600 mPa*s (cP) aufweist. Erfindungsgemäß vorteilhaft weist unter Standardbedingungen eine 0,25 %ige Lösung in Leitungswasser bei 3 rpm eine Viskosität von 600-1200 mPa*s (cP) auf. Erfindungsgemäß vorteilhaft hat das erfindungsgemäße Xanthangummi im Trockenzustand eine Partikelgröße 200 mesh. Erfindungsgemäß vorteilhaft kann beispielsweise Keltrol CG-F V von CP Kelco oder Xanthan Gum food grade, Type FF und Type FFPC von Jungbunzlauer eingesetzt werden.

Erfindungsgemäß vorteilhafte Ausführungsformen der vorliegenden Erfindung sind dadurch gekennzeichnet, dass die Zubereitung einen oder mehrere UV-Filter gewählt aus der Gruppe der Verbindungen 4-(tert.-Butyl)-4'-methoxydibenzoylmethan (INCI Butyl Methoxydibenzoyl-methane), (2-[-4-(Diethylamino)-2-hydroxybenzoyl] Benzoesäurehexylester (INCI: Diethyl-amino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) enthält.

Enthält die Zubereitung 4-(tert.-Butyl)-4'-methoxydibenzoylmethan (INCI Butyl Methoxydibenzoylmethane), so beträgt die erfindungsgemäß vorteilhafte 1 bis 5% Gew.-%, bezogen auf das Gesamtgewicht der Zubereitung.

Enthält die Zubereitung (2-[-4-(Diethylamino)-2-hydroxybenzoyl] Benzoesäurehexylester (INCI: Diethylamino Hydroxybenzoyl Hexyl Benzoate), so beträgt die erfindungsgemäß vorteilhafte Einsatzkonzentration von 1 bis 9 Gew.-%, bezogen auf das Gesamtgewicht der Zubereitung.

Enthält die Zubereitung 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine), so beträgt die erfindungsgemäß vorteilhafte Einsatzkonzentration von 0,5 bis Gew. 4,5 Gew.-% bezogen auf das Gesamtgewicht der Zubereitung.

Ferner sind erfindungsgemäß vorteilhafte Ausführungsformen dadurch gekennzeichnet, dass sie einen oder mehrere UV-Filter, gewählt aus der Gruppe der Verbindungen 2-Phenylbenzimidazol-5-sulfonsäuresalze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; Ethylhexylsalicylat; Terephthalidendicamphersulfonsäuresalze; 2-Ethylhexyl-2-cyano-3,3-diphenylacrylat; 4-(Dimethylamino)benzoesäure-amylester; 4-Methoxybenzalmalon-säuredi(2-ethylhexyl)ester; 4-Methoxyzimtsäureisoamylester; 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Piperazinderivate;Titandioxid; Zinkoxid, enthalten.

Dabei ist insbesondere der Einsatz von Phenylbenzimidazol-5-sulfonsäuresalzen, Ethylhexylsalicylat, Homomenthylsalicylat, 2-Ethylhexyl-2-cyano-3,3-diphenylacrylat, Dioctylbutylamidotriazon (INCI: Diethylhexyl-Butamidotriazone), 4,4',4"-(1,3,5-Triazin-2,4,6-triyltriimino)-tris-benzoësäure-tris(2-ethylhexylester) (INCI: Ethylhexyl Triazone), Titandioxid. erfindungsgemäß bevorzugt. Erfindungsgemäß besonders bevorzugt sind die UV-Filter Phenylbenzimidazol-5-sulfonsäuresalzen, Ethylhexylsalicylat, Dioctylbutylamidotriazon (INCI: Diethylhexyl-Butamidotriazone), 4,4',4"-(1,3,5-Triazin-2,4,6-triyltriimino)-tris-benzoësäure-tris(2-ethylhexylester) (INCI: Ethylhexyl Triazone).

Die erfindungsgemäß bevorzugten Einsatzkonzentrationen für Phenylbenzimidazol-5-sulfonsäuresalze beträgt von 0,5 bis 4 Gew.-%, bezogen auf das Gesamtgewicht der Zubereitung, die bevorzugte Einsatzkonzentration für Ethylhexylsalicylat beträgt von 0,5 bis 5 Gew.-%, bezogen auf das Gesamtgewicht.

Es ist vorteilhaft im Sinne der vorliegenden Erfindung, wenn die Zubereitung einen oder mehrere UV-Filter a) in einer Gesamtkonzentration von 1 bis 30 Gewichts-%, bezogen auf das Gesamtgewicht der Zubereitung, enthält.

Es ist erfindungsgemäß von Vorteil, wenn die Zubereitung hydriertes Rapsöl (INCI: hydrogenated rapeseed oil) enthält. Dieses kann beispielsweise unter dem Handelsnamen Dermofeel Viscolid pof bei der Firma Evonik Nutrition & Care GmbH oder der Dr. Straetmans GmbH Erworben werden.

Dabei ist erfindungsgemäß vorteilhaft, wenn die Zubereitung hydriertes Rapsöl (INCI: hydrogenated rapeseed oil) in einer Konzentration von 0,5 bis 3,0 Gewichts-%, bezogen auf das Gesamtgewicht der Zubereitung, enthält.

Vorteilhafte Ausführungsformen der vorliegenden Erfindung sind auch dadurch gekennzeichnet, dass die Zubereitung frei ist von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Parabenen (insbesondere Propyl- und Butylparaben), Methylisothiazolinon, Chlormethyl-isothiazolinon und DMDM-Hydantoin, Polyethylenglycolethern oder Polyethylenglycolestern.

Es ist erfindungsgemäß vorteilhaft, wenn die Zubereitung Hydroxypropylmethylcellulose in einer Konzentration von 0,05 bis 0,5 Gewichts-% und Xanthangummi in einer Konzentration von 0,1 bis 1 Gewichts-%, jeweils bezogen auf das Gesamtgewicht der Zubereitung enthält.

Es ist erfindungsgemäß bevorzugt, wenn die Zubereitung Hydroxypropylmethylcellulose in einer Konzentration von 0,1 bis 0,5 Gewichts-% und Xanthangummi in einer Konzentration von 0,3 bis 0,7 Gewichts-%, jeweils bezogen auf das Gesamtgewicht der Zubereitung enthält.

Vorteilhaft im Sinne der vorliegenden Erfindung ist es darüber hinaus, wenn das Gewichtsverhältnis von Hydroxypropylmethylcellulose zu Xanthangummi in der Zubereitung von 1:2 bis 2:7 beträgt.

Es ist erfindungsgemäß vorteilhaft, wenn die Zubereitung in Form einer O/W-Emulsion oder Hydrodispersion vorliegt.

Liegt die erfindungsgemäße Zubereitung in Form einer O/W-Emulsion vor, so ist sie erfindungsgemäß vorteilhaft dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Kaliumcetylphosphat, Polyglyceryl-10 Stearat, Natriumstearoylglutamat, enthält.

Es ist erfindungsgemäß vorteilhaft, wenn die Zubereitung Ethanol enthält. Ethanol wird erfindungsgemäß bevorzugt in einer Konzentration von 2 bis 10 Gewichts-%, bezogen auf das Gesamtgewicht der Zubereitung eingesetzt.

Vorteilhafte Ausführungsformen im Sinne der vorliegenden Erfindung sind dadurch gekennzeichnet, dass die Zubereitung Ethylhexylglycerin und/oder 4-Hydroxyacetophenon enthält.

Ferner ist es erfindungsgemäß von Vorteil, wenn die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; 8-Hexadecen-1,16-dicarbonsäure, Thiamidol, Glycerylglycose, (2-Hydroxyethyl)harnstoff, Vitamin E bzw. seine Derivate, Hyaluronsäure und/oder deren Salze und/oder Licochalcon A enthält.

Die erfindungsgemäße Zubereitung kann vorteilhaft Feuchthaltemittel enthalten. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel^{®}1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) und /oder Talkum.

Es ist erfindungsgemäß bevorzugt, wenn die erfindungsgemäße Zubereitung Tapiocastärke und/oder Distärkephosphat enthält.

Erfindungsgemäß vorteilhafte Ausführungsformen der vorliegenden Erfindung sind dadurch gekennzeichnet, dass die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Dabei ist es erfindungsgemäß bevorzugt, wenn die Zubereitung Dibutyladipat, Dicaprylylcarbonat, Butylene Glycol Dicaprylat/Dicaprat und/oder C12-C15 Alkylbenzoat enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Elektrolyte, Selbstbräuner etc.

Es ist ferner vorteilhaft im Sinne der vorliegenden Erfindung, wenn die erfindungsgemäße Zubereitung einen oder mehrere Parfümstoffe gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

Darüber hinaus können die erfindungsgemäßen Zubereitungen die für kosmetische Sonnenschutzmittel üblichen Inhaltsstoffe in den üblichen Einsatzkonzentrationen enthalten.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | |
|---|---|
| Aqua | 60.95 |
| Tocopheryl Acetate | 0.06 |
| Aqua + Trisodium EDTA | 0.50 |
| Phenoxyethanol | 0.40 |
| Dimethicone | 0.90 |
| C12-15 Alkyl Benzoate | 5.00 |
| Butyl Methoxydibenzoylmethane | 4.75 |
| Phenylbenzimidazole Sulfonic Acid | 0.50 |
| Behenyl Alcohol | 1.20 |
| Glycerin | 1.00 |
| Aqua + Sodium Hydroxide | 0.17 |
| Alcohol Denat. + Aqua | 7.50 |
| Ethylhexyl Salicylate | 2.00 |
| Xanthan Gum | 0.30 |
| Hydroxypropyl Methylcellulose | 0.20 |
| Ethylhexyl Triazone | 1.50 |
| Silica Dimethyl Silylate | 0.50 |
| Stearyl Alcohol | 1.20 |
| Bis-Ethylhexyloxyphenol Methoxyphenyl Triazine | 2.50 |
| Butylene Glycol Dicaprylate/Dicaprate | 2.00 |
| Glycyrrhiza Inflata Root Extract | 0.03 |
| Diethylamino Hydroxybenzoyl Hexyl Benzoate | 1.00 |
| Sodium Stearoyl Glutamate + Sodium Chloride | 0.25 |
| Titanium Dioxide (nano) + Silica + Dimethicone | 3.00 |
| Hydroxyacetophenone | 0.20 |
| Hydrogenated Rapeseed Oil | 2.00 |
| Parfum | 0.40 |
| | |
| **Summen:** | **100.00** |
| | |
| Isopropyl Palmitate | 6.00 |
| Aqua | 60.70 |
| Tocopheryl Acetate | 0.06 |
| Aqua + Trisodium EDTA | 0.50 |
| Phenoxyethanol | 0.40 |
| Dimethicone | 0.90 |
| C12-15 Alkyl Benzoate | 5.00 |
| Butyl Methoxydibenzoylmethane | 2.40 |
| Phenylbenzimidazole Sulfonic Acid | 0.50 |
| Behenyl Alcohol | 1.50 |
| Glycerin | 1.00 |
| Aqua + Sodium Hydroxide | 0.17 |
| Alcohol Denat. + Aqua | 7.50 |
| Ethylhexyl Salicylate | 2.00 |
| Xanthan Gum | 0.30 |
| Hydroxypropyl Methylcellulose | 0.20 |
| Ethylhexyl Triazone | 0.50 |
| Silica Dimethyl Silylate | 0.50 |
| Stearyl Alcohol | 1.50 |
| Bis-Ethylhexyloxyphenol Methoxyphenyl Triazine | 2.00 |
| Butylene Glycol Dicaprylate/Dicaprate | 2.00 |
| Glycyrrhiza Inflata Root Extract | 0.03 |
| Diethylamino Hydroxybenzoyl Hexyl Benzoate | 0.50 |
| Sodium Stearoyl Glutamate + Sodium Chloride | 0.25 |
| Titanium Dioxide (nano) + Silica + Dimethicone | 2.00 |
| Hydroxyacetophenone | 0.20 |
| Hydrogenated Rapeseed Oil | 1.00 |
| Parfum | 0.40 |
| | |
| **Summen:** | **100.00** |

## Patentansprüche

1. Kosmetische Zubereitung enthaltend
a) Hydroxypropylmethylcellulose und
b) Xanthangummi,
wobei die Zubereitung frei ist von Basis von Polyacrylaten, Carbomeren und Polyvinylpyrrolidonen, **dadurch gekennzeichnet, dass** das Gewichtsverhältnis von Hydroxypropylmethylcellulose zu Xanthangummi in der Zubereitung von 1:2 bis 2:7 beträgt.

2. Verfahren zur Viskositätsregulierung Polyacrylat-freier kosmetischer Zubereitungen, **dadurch gekennzeichnet, dass** der Zubereitung eine Mischung aus Hydroxypropylmethylcellulose und Xanthangummi zugesetzt wird.

3. Verwendung einer Mischung aus Hydroxypropylmethylcellulose und Xanthangummi als Ersatzstoff für Polyacrylate in Polyacrylat-freien kosmetischen Zubereitungen.

4. Kosmetische Zubereitung nach Anspruch 1, Verfahren nach Anspruch 2 oder Verwendung nach Anspruch 3, **dadurch gekennzeichnet, dass** die Zubereitung einen oder mehrere UV-Filter gewählt aus der Gruppe der Verbindungen 4-(tert.-Butyl)-4'-methoxydibenzoylmethan (INCI Butyl Methoxydibenzoyl-methane), (2-[-4-(Diethylamino)-2-hydroxybenzoyl] Benzoesäurehexylester (INCI: Diethyl-amino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) enthält.

5. Kosmetische Zubereitung, Verfahren oder Verwendung nach einem der vorhergehenden Ansprüche, **dadurch gekennzeichnet, dass** die Zubereitung hydriertes Rapsöl (INCI: hydrogenated rapeseed oil) enthält.

6. Kosmetische Zubereitung, Verfahren oder Verwendung nach einem der vorhergehenden Ansprüche, **dadurch gekennzeichnet, dass** die Zubereitung frei ist von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Parabenen (insbesondere Propyl- und Butylparaben), Methylisothiazolinon, Chlormethyl-isothiazolinon und DMDM-Hydantoin, Polyethylenglycolethern oder Polyethylenglycolestern.

7. Kosmetische Zubereitung, Verfahren oder Verwendung nach einem der vorhergehenden Ansprüche, **dadurch gekennzeichnet, dass** die Zubereitung Hydroxypropylmethylcellulose in einer Konzentration von 0,05 bis 0,5 Gewichts-% und Xanthangummi in einer Konzentration von 0,1 bis 1 Gewichts-%, jeweils bezogen auf das Gesamtgewicht der Zubereitung enthält.

8. Verfahren oder Verwendung nach einem der vorhergehenden Ansprüche, **dadurch gekennzeichnet, dass** das Gewichtsverhältnis von Hydroxypropylmethylcellulose zu Xanthangummi in der Zubereitung von 1:2 bis 2:7 beträgt.

9. Kosmetische Zubereitung, Verfahren oder Verwendung nach einem der vorhergehenden Ansprüche, **dadurch gekennzeichnet, dass** die Zubereitung hydriertes Rapsöl (INCI: hydrogenated rapeseed oil) in einer Konzentration von 0,5 bis 3 Gewichts-%, bezogen auf das Gesamtgewicht der Zubereitung, enthält.

10. Kosmetische Zubereitung, Verfahren oder Verwendung nach einem der vorhergehenden Ansprüche, **dadurch gekennzeichnet, dass** die Zubereitung in Form einer O/W-Emulsion oder Hydrodispersion vorliegt.

11. Kosmetische Zubereitung, Verfahren oder Verwendung nach einem der vorhergehenden Ansprüche, **dadurch gekennzeichnet, dass** die Zubereitung in Form einer O/W-Emulsion oder Hydrodispersion vorliegt.

## Claims

1. Cosmetic preparation comprising
a) hydroxypropyl methylcellulose and
b) xanthan gum,
wherein the preparation is free from polyacrylates, carbomers and polyvinylpyrrolidones, **characterized in that** the ratio by weight of hydroxypropyl methylcellulose to xanthan gum in the preparation is from 1:2 to 2:7.

2. Method for regulating the viscosity of polyacrylate-free cosmetic preparations, **characterized in that** a mixture of hydroxypropyl methylcellulose and xanthan gum is added to the preparation.

3. Use of a mixture of hydroxypropyl methylcellulose and xanthan gum as a substitute for polyacrylates in polyacrylate-free cosmetic preparations.

4. Cosmetic preparation according to Claim 1, the method according to Claim 2 or the use according to Claim 3, **characterized in that** the preparation comprises one or more UV filters selected from the group of compounds comprising 4-(tert-butyl)-4'-methoxydibenzoylmethane (INCI Butyl Methoxydibenzoylmethane), hexyl (2-[4-(diethylamino)-2-hydroxybenzoyl]benzoate (INCI: Diethylamino Hydroxybenzoyl Hexyl Benzoate) and 2,4-bis{[4-(2-ethylhexyloxy)-2-hydroxy]phenyl}-6-(4-methoxyphenyl)-1,3,5-triazine (INCI Bis-Ethylhexyloxyphenol Methoxyphenyl Triazine).

5. Cosmetic preparation, method or use according to any of the preceding claims, **characterized in that** the preparation comprises hydrogenated rapeseed oil (INCI: Hydrogenated Rapeseed Oil).

6. Cosmetic preparation, method or use according to any of the preceding claims, **characterized in that** the preparation is free from 3-(4-methylbenzylidene)camphor, 2-hydroxy-4-methoxybenzophenone (INCI: Oxybenzone), 2-ethylhexyl 4-methoxycinnamate (INCI Octyl Methoxycinnamate), parabens (particularly propyl and butyl paraben), methylisothiazolinone, chloromethylisothiazolinone and DMDM hydantoin, polyethylene glycol ethers or polyethylene glycol esters.

7. Cosmetic preparation, method or use according to any of the preceding claims, **characterized in that** the preparation comprises hydroxypropyl methylcellulose at a concentration of 0.05 to 0.5% by weight and xanthan gum at a concentration of 0.1 to 1% by weight, based in each case on the total weight of the preparation.

8. Method or use according to any of the preceding claims, **characterized in that** the ratio by weight of hydroxypropyl methylcellulose to xanthan gum in the preparation is from 1:2 to 2:7.

9. Cosmetic preparation, method or use according to any of the preceding claims, **characterized in that** the preparation comprises hydrogenated rapeseed oil (INCI: Hydrogenated Rapeseed Oil) at a concentration of 0.5 to 3% by weight, based on the total weight of the preparation.

10. Cosmetic preparation, method or use according to any of the preceding claims, **characterized in that** the preparation is in the form of an O/W emulsion or hydrodispersion.

11. Cosmetic preparation, method or use according to any of the preceding claims, **characterized in that** the preparation is in the form of an O/W emulsion or hydrodispersion.

## Revendications

1. Préparation cosmétique contenant
a) de l'hydroxypropylméthylcellulose et
b) de la gomme xanthane,
la préparation ne contenant pas de base de polyacrylates, de carbomères et de polyvinylpyrrolidones, **caractérisée en ce que** le rapport en poids de l'hydroxypropylméthylcellulose à la gomme xanthane dans la préparation est de 1:2 à 2:7.

2. Procédé de régulation de la viscosité de préparations cosmétiques sans polyacrylates, **caractérisé en ce que** la préparation est additionnée d'un mélange d'hydroxypropylméthylcellulose et de gomme xanthane.

3. Utilisation d'un mélange d'hydroxypropylméthylcellulose et de gomme xanthane comme substitut de polyacrylates dans des préparations cosmétiques sans polyacrylates.

4. Préparation cosmétique selon la revendication 1, procédé selon la revendication 2 ou utilisation selon la revendication 3, **caractérisé en ce que** la préparation contient un ou plusieurs filtres UV choisis dans le groupe des composés 4-(tert-butyl)-4'-méthoxydibenzoylméthane (INCI Butyl Methoxydibenzoyl-methane), ester hexylique de l'acide (2-[-4-(diéthylamino)-2-hydroxybenzoyl] benzoïque (INCI : Diethyl-amino Hydroxybenzoyl Hexyl Benzoate) et 2,4-bis-{[4-(2-éthyl-hexyloxy)-2-hydroxy]-phényl}-6-(4-méthoxyphényl)-1,3,5-triazine (INCI Bis-Ethylhexyloxyphenol Methoxyphenyl Triazine).

5. Préparation cosmétique, procédé ou utilisation selon l'une des revendications précédentes, **caractérisé en ce que** la préparation contient de l'huile de colza hydrogénée (INCI : hydrogenated rapeseed oil).

6. Préparation cosmétique, procédé ou utilisation selon l'une des revendications précédentes, **caractérisé en ce que** la préparation ne contient pas de 3-(4-méthylbenzylidène)-camphre, de 2-hydroxy-4-méthoxybenzophénone (INCI : Oxybenzon), de l'ester 2-éthylhexylique de l'acide 4-méthoxycinnamique (INCI Octylmethoxycinnamate), de parabens (en particulier le propyl- et le butylparaben), de méthylisothiazolinone, de chlorométhyl-isothiazolinone et de DMDM-hydantoïne, de polyéthylèneglycoléthers ou de polyéthylèneglycolesters.

7. Préparation cosmétique, procédé ou utilisation selon l'une des revendications précédentes, **caractérisé en ce que** la préparation contient de l'hydroxypropylméthylcellulose à une concentration de 0,05 à 0,5 % en poids et de la gomme xanthane à une concentration de 0,1 à 1 % en poids, dans chaque cas par rapport au poids total de la préparation.

8. Procédé ou utilisation selon l'une des revendications précédentes, **caractérisé en ce que** le rapport en poids de l'hydroxypropylméthylcellulose à la gomme xanthane dans la préparation est de 1:2 à 2:7.

9. Préparation cosmétique, procédé ou utilisation selon l'une des revendications précédentes, **caractérisé en ce que** la préparation contient de l'huile de colza hydrogénée (INCI : hydrogenated rapeseed oil) à une concentration de 0,5 à 3 % en poids par rapport au poids total de la préparation.

10. Préparation cosmétique, procédé ou utilisation selon l'une des revendications précédentes, **caractérisé en ce que** la préparation se présente sous forme d'une émulsion huile-dans-l'eau ou d'une hydrodispersion.

11. Préparation cosmétique, procédé ou utilisation selon l'une des revendications précédentes, **caractérisé en ce que** la dispersion se présente sous forme d'une émulsion huile-dans-l'eau ou d'une hydrodispersion.
